Europäisches Patentamt

(19) European Patent Office

Office européen des brevets

(11) Publication number: **0 362 209 B1**

# (12) EUROPEAN PATENT SPECIFICATION

(45) Date of publication of patent specification: **08.06.94**

(51) Int. Cl.⁵: **C07K 7/10**, A61K 37/02, C07K 7/08

(21) Application number: **88903010.2**

(22) Date of filing: **23.02.88**

(86) International application number: **PCT/US88/00520**

(87) International publication number: **WO 88/06597 (07.09.88 88/20)**

(54) NEW SYNTHETIC BIOACTIVE COMPOUNDS AND METHOD OF PRODUCING BIOACTIVE EFFECT.

(30) Priority: **04.03.87 US 21493**
**23.07.87 US 76734**
**05.08.87 US 81793**

(43) Date of publication of application:
**11.04.90 Bulletin 90/15**

(45) Publication of the grant of the patent:
**08.06.94 Bulletin 94/23**

(84) Designated Contracting States:
**AT BE CH DE FR GB IT LI LU NL SE**

(56) References cited:

The Journal of Biol. Chemistry, vol. 261, no. 12, 25 April 1986, B. Gibson, pp. 5341-5349

Biochem. 149, 1985, Andreu, pp. 531-535

Ann. Rev. Biochem., 53, 1984, D. Eisenberg, pp. 545-623

(73) Proprietor: **THE UNITED STATES OF AMERICA as represented by the Secretary United States Department of Commerce National Technical Information Service, Office of Government Inventions and Patents,**
**5285 Port Royal Road**
**Springfield, Virginia 22161(US)**

(72) Inventor: **ZASLOFF, Michael, Alan**
**14431 Woodcrest Drive**
**Rockville, MD 20853(US)**

(74) Representative: **Lamb, John Baxter et al**
**MARKS & CLERK,**
**57-60 Lincoln's Inn Fields**
**London WC2A 3LS (GB)**

Proc. Natl. Acad. Sci. USA, vol. 81, Jan. 1984, I. Sures, pp. 380-384

The Journal of Biol. Chemistry, vol. 257, no. 5, 10 March 1982, C. Kempf, pp. 2469-2476

Proc. Nat. Acad. Sci. USA, vol. 80, Febr. 1983, E. Kaiser, pp. 1137-1143

Nature, vol. 292, 16 July 1981, H. Steiner, pp. 246-248

Biochemistry, 21, 1982, R. Merrifield, pp. 5020-5031

Toxicon, vol. 7, 1969, A. Csordas, pp. 103-108

Peptides, vol. 6, suppl. 3, 1985, K. Richter, pp. 17-21

Biochem. J., 243, 1987, M.G. Giovannini, pp. 113-120

The EMBO Journal, vol. 2, no. 5, 1983, W. Hoffmann, pp. 711-714

**Description**

BACKGROUND OF THE INVENTION

The present invention relates generally to bioactive compounds and a new method of producing bioactive effect. More particularly, the present invention is related to a new class of synthetic polypeptides, designated herein "Magainins", which have a broad range of bioactive properties. The new method of producing bioactive effect comprises, contacting a subject susceptible to microbial invasion or contamination with antimicrobial amount of Magainins, XPF or PGLa polypeptides.

Prior Art

Although "Magainin" family of compounds having the properties as described herein have never heretofore been discovered or known, XPF and PGLa polypeptides have been described in the literature and are charaterized by the following amino acid sequence:

PGLa:    GMASKAGAIAGKIAKVALKAL (NH$_2$)
XPF:    GWASKIGQTLGKIAKVGLKELIQPK

A review of XPF and PGLa can be found in Hoffman, et al. EMBO J., 2:711-714 (1983); Andreu, et al., J. Biochem, 149:531-535 (1985); Gibson, et al., J. Biol. Chem., 261:5341-5349 (1986); and Giovannini, et al., Biochem. J., 243:113-120 (1987). It should be noted that no function or useful property of these peptides has been identified in the literature.

SUMMARY OF THE INVENTION

It is, therefore, an object of the present invention to provide a novel family of synthetic compounds, termed "Magainins."

It is a further object of the present invention to provide a novel class of antimicrobial compounds capable of inhibiting the growth or proliferation of such organisms as gram-positive and gram-negative bacteria, fungi, virus, protozoan species and the like.

It is another object of the present invention to provide a new use of a known compound.

It is a still further object of the present invention to provide a method of controlling, preventing or eradicating microbial growth or proliferation by contacting the microbes with antimicrobial amount of XPF or PGLa polypeptides.

It is yet another object of the present invention to provide a method of producing bioactive effect employing XPF and PGLa in bioactive amounts.

Various other objects and advantages will become apparent from the detailed description of the invention.

DETAILED DESCRIPTION OF THE INVENTION

The above and various other objects and advantages of the present invention are achieved by Magainins which are defined as a class of substantially pure, homogeneous polypeptides composed exclusively of about 25 amino acids, having a molecular weight of about 2500 or less, being water soluble at a concentration of greater than 5mg per ml at neutral pH or in an aqueous solution of physiologic ionic strength, being amphiphilic, surface-seeking and having a broad spectrum of properties at physiologic ionic strength and pH. The Magainins are distinguishable from all other peptides reported in the literature at least with respect to their amino acid sequence. By computer search no analogous peptide with significant homology was found in the NIH protein and nucleic acid Data Banks (GenBank, 1986 Edition). Furthermore, the Magainins cannot be generated through simple substitutions of peptides of other classes with similar properties such as cecropin, xenopsin, bombesin, mellitin and the like. Hence, Magainins represent a unique family of chemical substances, clearly defined at least in part by their unique amino acid sequence.

Table I shows the amino acid sequence (single letter code) of certain Magainin polypeptides of the present invention.

TABLE I

| Primary Sequence of Magainin Polypeptides | |
|---|---|
| Magainin I: | $(NH_2)$GIGKFLHSAGKFGKAFVGEIMKS(OH) |
| Maganin II: | $(NH_2)$GIGKFLHSAKKFGKAFVGEIMNS(OH) |
| Magainin III: | $(NH_2)$GIGKFLHSAKKFGKAFVGEIMN(OH) |

Certain other objects of the present invention are the use of XPF, PGLa and/or a magainin, as herein defined for the manufacture of a medicament for the treatment or control of a microbial infection.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned hereunder are incorporated herein by reference.

The term "bioactive" as used herein means having biological effect such as illustrated herein infra.

The term "antibiotic" as used herein means that the polypeptides of the present invention produce effects adverse to the normal biological functions of the cell, tissue or organism including death or destruction and prevention of the growth or proliferation of the biological system.

The term "antimicrobial" as used herein means that the polypeptides of the present invention inhibit, prevent or destroy the growth or proliferation of microbes such as bacteria, fungi, virus and the like.

The term "substantially pure" as used herein means as pure as it is possible to obtain by using the methods known to one of ordinary skill in the art.

Of course, having described the amino acid sequence of the Magainins, these polypeptides can be routinely synthesized in substantially pure form by standard techniques well known in the art, such as by commercially available peptide synthesizers and the like. Such standard techniques of polypeptide synthesis can be found described in such publications as Merrifield, J. Chem. Soc., 85:2149-2154 (1963); Hunkapillar, et al., Nature, 310:105-111 (1984).

It is noted, however, that the amino acid sequences of Magainins listed in Table I show only essential portions required for antibiotic activity. In otherwords, the polypeptides are not limited to the sequences shown in Table I, but must have, the amino acid sequence shown in Tables I and II. Some derivatives of Magainin II and their antimicrobial activities, for instance, are shown in Table II.

4

Table III shows the antimicrobial spectrum of the synthetic Magainin peptides of the present invention.

## TABLE II

### Antimicrobial Activity of Truncated Synthetic Derivatives of Magainin II and Other Peptides

| Derivative (Single Letter Code) | Zone of Inhibition on Lawn of E. coli Y1088 Generated by 50ug of the Peptide (mm) |
|---|---|
| Magainin II(a)  $(NH_2)$ IGKFLHSAKKFGKAFVGEIMNS(OH) | 10 |
| Magainin II(b)  $(NH_2)$ GKFLHSAKKFGKAFVGEIMNS(OH) | 10 |
| Magainin II(c)  $(NH_2)$ KFLHSAKKFGKAFVGEIMNS(OH) | 10 |
| Magainin II(d)  $(NH_2)$ FLHSAKKFGKAFVGEIMNS(OH) | 2 |
| Magainin II(e)  $(NH_2)$ LHSAKKFGKAFVGEIMNS(OH) | 1 |
| Magainin II(f)  $(NH_2)$ HSAKKFGKAFVGEIMNS(OH) | 0 |
| *Magainin II(g)  $(NH_2)$ GIGKFLHSAKKFGKAFVGEIMNS$(NH_2)$ | 15 |
| PGLa | 10 |
| XPF $(NH_2)$ | 15 |

* prior art compound

## TABLE III

### Antimicrobial Spectrum of Synthetic Magainin Peptides

| Organism(ATCC) | Magainin I | Magainin II | Magainin III |
| --- | --- | --- | --- |
| (Minimal Inhibitory Concentration (µg/ml) | | | |
| Eschericia coli D31 | 1-20 | 1-10 | 1-10 |
| Acinetobacter caloaceticus(19605) | 7-35 | 1-7 | 7-35 |
| Shigella sonnei (25931) | 7-35 | 1-7 | 7-35 |
| Enterobacter sonnei (23355) | 7-35 | 1-7 | 7-35 |
| Eschericia coli (25922) | 7-35 | 1-7 | 7-35 |
| Streptococcus pyogenes (19615) | 7-35 | 1-7 | 7-35 |
| Shigella flexneri (12022) | 35-70 | 7-35 | 7-35 |
| Citrobacter freundii (8090) | >100 | 7-35 | 7-35 |
| Enterobacter aerogenes (13048) | >100 | 7-35 | >100 |
| Klebisiella pneumonia (13883) | >100 | 7-35 | >100 |
| Staphyloccus epidermidis (1228) | >100 | 7-35 | 35-70 |

6

## TABLE III (Continued)

### Antimicrobial Spectrum of Synthetic Magainin Peptides

| Organism(ATCC) | Magainin I | Magainin II | Magainin III |
| --- | --- | --- | --- |
| (Minimal Inhibitory Concentration (µg/ml) | | | |
| Streptococcus faecalis (19433) | >100 | 35-70 | >100 |
| Pseudomonas aeruginosa (27853) | >100 | 35-70 | 35-70 |
| Salmonella typhimurium (14028) | >100 | 35-70 | 35-70 |
| Staphylococcus aureus (25923) | >100 | 35-70 | >100 |
| Candida albicans (14053) | >100 | 35-70 | >100 |
| Proteus vulgaris (13315) | >100 | >100 | >100 |
| Serratia marcescens (8100) | >100 | >100 | >100 |

Table III also lists the range of minimal inhibitory concentrations for various organisms assayed in trypticase soy broth. These determinations were made following standard techniques well known in the art and described in such publications as, DIFCO Manual, 10th edition, 1984, pages 78-80.

Table IV lists the approximate concentration of Magainins which induces physical lysis of several representative protozoan species. These determinations where made by visual assessment of the treated organisms by light microscopy. Of course, the optimal effective antimicrobial concentration of a particular Magainin can be easily determined in a routine manner without undue experimentation by techniques well known to one of ordinary skill in the art.

It is clear from the results presented herein supra that the Magainin polypeptides of the present invention have a broad range of potent biological activity including antimicrobial potency. Hence, it is apparent that the Magainins of the present invention allow a method of treating or controlling microbial infection caused by those organisms which are sensitive to Magainin. Such treatment comprises administering to a host or tissue susceptible to or afflicted with microbial infection of an antimicrobial amount of Magainin.

Clearly, due to their antibiotic properties, the Magainins of the present invention can also be used as preservatives or sterilants of materials susceptible to microbial contamination.

7

EP 0 362 209 B1

TABLE IV

| Antiprotozoan Activity of Synthetic Magainin Peptides | | | |
|---|---|---|---|
| Organism | Magainin I | Magainin II | Magainin III |
| (Concentration required for osmotic lysis (μg/ml)) | | | |
| Paramecium caudatum | 5 | 5 | 5 |
| Amoeba proteus | 5 | 5 | 5 |
| Euglena gracilis | 5 | 5 | 5 |

The substitution of phenylalanine at residue 5 by tyrosine results in a slight decrease in MIC of the derivative compared to Magainin II; however, a compensatory advantage of the tyrosine residue allows coupling of radioactive moieties such as 125 I and the like with the polypeptide molecule so that such radioactive derivatives can be employed in standard radio-immunoassay procedures to determine Magainin levels.

Anti-Protozoan Activity:

Magainin II(g), a prior art compound, has been found to be active against the Malarial parasite, Plasmodium gallinaceum. Observations of activity were conducted by direct light microscopy. A sample of female and male zygotic forms of the chicken malarial parasite were suspended in phosphate buffered saline. Magainin II(g) was added to each at 100 ug/ml. Within seconds both forms underwent evident physical lysis. A control consisting of the addition of Magainin II(f) had no visible effect. The susceptibility of this strain of malaria suggests that the Magainins may be utilized as an anti-malarial agent in human Plasmodium infections.

It may be noted that although at optimum antimicrobial concentrations, Magainins are not cytotoxic, however, at higher concentrations Magainins exhibit cytotoxic activity. This activity is considered a significant attribute of the Magainin peptides in that it permits their use of the killing of animal cells and therefore in the utility of Magainins as therapeutic cytotoxic agents. This includes anti-tumor activity, in vivo cytolysis or growth inhibition of malignant cells, such as in leukemias and selective reduction in specific cell populations (e.g., removal of lymphocytes from blood).

Cells were suspended in standard minimal Eagles medium containing 10% fetal calf serum. VERO cells and KB cells were obtained from the American Type Culture Collection. Human diploid fibroblasts were propagated from a primary dermal biopsy. Human lymphocytes were collected from a buffy coat preparation by centrifugation in ficoll-hyapaque medium. Human buccal epithelium was obtained by scraping the oral mucosa with a glass cover-slip and resuspending the cellular scraping in MEM. Cytotoxicity was determined by loss of Trypan Blue exclusion, following addition of dye to the medium. Cytotoxicity was assessed 30 minutes after addition of the peptide. Concentration noted represents the concentration of peptide resulting in 50% toxicity as determined visually by light microscopic examination of the treated cells.

Spermicidal Activity of Magainin Peptides

The activity of Magainin II(g), a prior art compound, was determined against human sperm since the peptides were shown to exhibit cytotoxic activity against a variety of human cells. Magainin II(g) was added in various concentrations to a suspension of human sperm in phosphate buffered saline. Motility was assayed by visual inspection under a light microscope. Observations were conducted at room temperature (about 22°-25°C). The approximate number of motile sperm observed in 20 independent fields was noted as a function of concentration. Magainin II(g) inhibits sperm motility by 50% at 35 ug/ml and by 100% at 75 ug/ml. These results indicate that Magainin II(g) and other derivatives are useful as spermicide and hence, as contraceptive agent. The association of anti-microbial activity of the Magainin peptides along with its spermicidal activity indicates that this family of peptides represents ideal spermicidal agents.

Anti-Viral Activity of Magainin Peptides

The anti-viral activity of Magainin was tested against the presumptive AIDS virus, HTLV III in vitro and has been found in three repeated studies to exhibit anti-viral activity. HTLV III was obtained from culture

8

media from the producing cell line A.3101. The media was diluted with MEM containing 10% fetal calf serum (FCS) to a viral concentration of about $10^4$ pfu/ml. Magainin II(g) was added to the viral inoculum (1 ml) to a concentration of 200 ug/ml and incubated at 37°C for 30 min. The viral innoculum was then added to a well containing $10^3$ tester cells (cell line, SUPT1) and 1 ml of MEM with 10% FCS. Viral activity was assessed by formation of syncytia and cell death in the test cell culture as compared with cell cultures unexposed to Magainin peptides. After an incubation period of 2 weeks Magainin-exposed cultures appeared identical to cultures not exposed to virus, while untreated cultures were fully destroyed. Assay of reverse transcriptase activity in the culture fluids of untreated and created wells revealed that Magainin treated samples exhibited no greater reverse transcriptase activity than uninfected cells, corroborating the visual assessment of cellular protection.

These studies demonstrate that the Magainin peptides have the potential to reduce the infectivity of the AIDS virus, HTLV III. The use of the Magainin peptides as a sterilant against this virus or as a chemotherapeutic agent for treating AIDS is thus indicated.

The activity of the Magainin peptides against HTLV III in vitro suggests that these peptides will exhibit anti-viral activity against other viral agents. The property of the Magainin peptides to disrupt certain membrane structures leads to the obvious application of these peptides against other membrane enclosed viruses, of which HTLV III is only a representative organism. Other candidates include herpes viruses, hepatitis B virus, retroviruses such as feline leukemia virus and HTLV I, and influenza virus.

Of course, pharmaceutical compositions comprising Magainin polypeptides of the present invention as an active ingredient in an amount sufficient to produce antibiotic effect in susceptible host or tissue and a pharmaceutically acceptable, non-toxic sterile carrier can be easily prepared based on the data provided herein. Such carriers could be fillers, non-toxic buffers, physiological saline solution and the like. The preparation can be used topically or systemically and may be in any suitable form such as liquid, solid or semi-solid, which include injectable solutions, tablets, ointments, lotions, pastes, capsules and the like. Of course, the Magainins may also be adminstered in combination with other adjuvants, protease inhibitors, or compatible drugs where such combination is seen desirable or advantageous in controlling the infection caused by harmful microorganisms including protozoa, viruses and the like.

Table V shows the minimal inhibitory concentrations of XPF, PGLa and Magainin II(g) against different bacteria and fungi. As can be appreciated, the relative potency of each of the peptides differs somewhat with respect to a particular organism, but the activity is quite similar.

Table VI lists the minimal effective concentrations of XPF, PGLa and Magainin II(g) necessary to physically disrupt several different species of protozoa. In each case, the peptide induces osmotic swelling indicating a common mechanism.

TABLE V

| Spectrum of Antimicrobial Activity of Synthetic Peptides | | | |
|---|---|---|---|
| ORGANISM | MINIMAL INHIBITORY CONCENTRATION ($\mu$g/ml) | | |
| | Magainin II(g) | PGLa | XPF |
| E. coli (25922) | 10-50 | 10-50 | 10-50 |
| P. aeruginosa (27883) | 50-100 | 200-500 | 50-100 |
| S. aureus (250230 | 500 | 50-100 | 100-200 |
| S. pyogenes (19615) | 10-50 | 10 | 10-50 |
| S. cerviscae ( ) | 50-100 | 100-200 | 100-200 |
| C. albicans (14053) | 200-500 | 100-200 | 200-500 |

$10^5$ Organisms were inoculated from late-log phase cultures into 0.5 ml of TSB broth containing synthetic peptide at concentrations of 10, 50, 100, 200 and 500 $\mu$g/ml. Cultures were incubated at either 37°C (bacteria) or 30°C (fungi) for 24 hours. Concentrations of peptide at which no visible growth of innoculum occurred is listed.

TABLE VI

| Sensitivity of Protozoa to Synthetic Peptides | | | |
|---|---|---|---|
| ORGANISM | MINIMAL DISRUPTIVE CONCENTRATION ($\mu$g/ml) | | |
| | Magainin II | PGLa | XPF |
| Paramecium caudatum | 10 | 5 | 10 |
| Tetrahymena pyriformis | 20 | 20 | 20 |
| Acanthameoba castellani | 2 | 2 | 2 |

Protozoa (about $10^2$) were suspended into 200 $\mu$l of 1% trypticase soy broth on a glass depression slide. Actively growing cultures of P. caudatum and T. pyrifomis were obtained from Nasco (Wisc) and A. castellani from B Bowers (NHLBI). Peptides were added to various concentrations and the organism visually assessed by light microscopy within 15 minutes of exposure. The concentrations listed represent the minimal peptide concentration at which physical disruption of all protozoa occurred.

It is clear from the results presented herein that XPF and PGLa are potent antimicrobial agents.

It is understood that the examples and embodiments described herein are for illustrative purposes only and that various modifications or changes in light thereof will be suggested to persons skilled in the art and are to be included within the spirit and purview of this application and the scope of the appended claims.

**Claims**

1. A Magainin having an amino acid sequence of (NH$_2$)GIGKFLHSAGKFGKAFVGEIMKS(OH).

2. A Magainin having an amino acid sequence of (NH$_2$)GIGKFLHSAKKFGKAFVGEIMNS(OH).

3. A Magainin having an amino acid sequence of (NH$_2$)GIGKFLHSAKKFGKAFVGEIMN(OH).

4. A Magainin having an amino acid sequence of (NH$_2$)IGKFLHSAKKFGKAFVGEIMNS(OH).

5. A Magainin having an amino acid sequence of (NH$_2$)GKFLHSAKKFGKAFVGEIMNS(OH).

6. A Magainin having an amino acid sequence of (NH$_2$)KFLHSAKKFGKAFVGEIMNS(OH).

7. A Magainin having an amino acid sequence of (NH$_2$)FLHSAKKFGKAFVGEIMNS(OH).

8. A Magainin having an amino acid sequence of (NH$_2$) LHSAKKFGKAFVGEIMNS(OH).

9. A pharmaceutical composition comprising, as an active ingredient, a Magainin according to any one of claims 1 to 8, together with a pharmaceutically acceptable diluent or carrier therefor.

10. Use of XPF, PGLa and/or a Magainin according to any one of the claims 1 to 8 for the manufacture of a medicament for the treatment or control of microbial infection.

**Patentansprüche**

1. Ein Magainin einer Aminosäuresequenz von (NH$_2$)GIGKFLHSAGKFGKAFVGEIMKS (OH).

2. Ein Magainin einer Aminosäuresequenz von (NH$_2$)GIGKFLHSAKKFGKAFVGEIMNS (OH).

3. Ein Magainin einer Aminosäuresequenz von (NH$_2$)GIGKFLHSAKKFGKAFVGEIMN (OH).

4. Ein Magainin einer Aminosäuresequenz von (NH$_2$)IGKFLHSAKKFGKAFVGEIMNS (OH).

5. Ein Magainin einer Aminosäuresequenz von (NH$_2$)GKFLHSAKKFGKAFVGEIMNS (OH).

6. Ein Magainin einer Aminosäuresequenz von (NH$_2$)KFLHSAKKFGKAFVGEIMNS (OH).

**7.** Ein Magainin einer Aminosäuresequenz von (NH$_2$)FLHSAKKFGKAFVGEIMNS (OH).

**8.** Ein Magainin einer Aminosäuresequenz von (NH$_2$)LHSAKKFGKAFVGEIMNS (OH).

**9.** Eine pharmazeutische Zusammensetzung, enthaltend als aktiven Bestandteil ein Magainin nach einem der Ansprüche 1 bis 8 gemeinsam mit einem dafür geeigneten pharmazeutischen Verdünnungsmittel oder Träger.

**10.** Verwendung von XPF, PGLa und/oder einem Magainin nach einem der Ansprüche 1 bis 8 zur Herstellung eines Arzneimittels zur Behandlung oder zur Kontrolle einer mikrobiellen Infektion.

**Revendications**

**1.** Un polypeptide synthétique dénommé magainine ayant la séquence d'aminoacides suivante :
(NH$_2$) GIGKFLHSAGKFGKAFVGEIMKS (0H).

**2.** Une magainine ayant la séquence d'aminoacides suivante :
(NH$_2$) GIGKFLHSAKKFGKAFVGEIMNS (OH).

**3.** Une magainine ayant la séquence d'aminoacides suivante :
(NH$_2$) GIGKFLHSAKKFGKAFVGEIMN (OH).

**4.** Une magainine ayant la séquence d'aminoacides suivante :
(NH$_2$) IGKFLHSAKKFGKAFVGEIMNS (OH).

**5.** Une magainine ayant la séquence d'aminoacides suivante :
(NH$_2$) GKFLHSAKKFGKAFVGEIMNS (OH).

**6.** Une magainine ayant la séquence d'aminoacides suivante :
(NH$_2$) KFLHSAKKFGKAFVGEIMNS (OH).

**7.** Une magainine ayant la séquence d'aminoacides suivante :
(NH$_2$) FLHSAKKFGKAFVGEIMNS (OH).

**8.** Une magainine ayant la séquence d'aminoacides suivante :
(NH$_2$) LHSAKKFGKAFVGEIMNS (OH).

**9.** Une composition pharmaceutique comprenant, comme ingrédient actif, une magainine selon l'une quelconque des revendications 1 à 8, ensemble avec un diluant ou support pharmaceutiquement acceptable.

**10.** Utilisation du XPF, PGla et/ou d'une magainine selon l'une quelconque des revendications 1 à 8 pour la fabrication d'un médicament pour le traitement ou le contrôle d'une infection microbienne.